# EUROPÄISCHE PATENTSCHRIFT

(11) **EP 0 434 008 B1**
(45) Veröffentlichungstag und Bekanntmachung des Hinweises auf die Patenterteilung: **22.06.1994**
(21) Anmeldenummer: 90124649.6
(22) Anmeldetag: 18.12.1990
(51) Int. Cl.: A61M 5/32

(54) **Schutzvorrichtung für Injektionsnadeln**
Injection needle protection device
Dispositif de protection pour aiguille à injection

(30) Priorität: 18.12.1989 YU 2397/89
(43) Veröffentlichungstag der Anmeldung: 26.06.1991
(73) Patentinhaber: Lazovski, Tase, YU-65000 Nova Gorica (YU)
(72) Erfinder: Lazovski, Tase, YU-65000 Nova Gorica (YU)
(74) Vertreter: Finck, Dieter, Dr.Ing.

(56) Entgegenhaltungen:
- EP-A- 0 229 204
- WO-A-89/10767
- DE-A- 3 802 353
- US-A- 4 725 267
- US-A- 4 775 369

## Beschreibung

Die Erfindung betrifft eine Schutzvorrichtung für eine Injektionsnadel gemäß dem ersten Teil von Anspruch 1.

Bei einer solchen, aus der US-A-4 725 267 bekannten Schutzvorrichtung ist die Injektionsnadel in der Bohrung in einem freien Schiebesitz geführt. Das Wendelelement ist eine Schraubendruckfeder, die sich mit einem Ende an dem Sockelteil der Injektionsnadel abstützt, während ihr anderes Ende an dem Anschlagbund angreift. Die Hülse erstreckt sich außen um die Schraubendruckfeder herum und ist lösbar an dem Sockelteil oder einem ringförmigen Fassungsteil der Injektionsnadel festgelegt. Diese Festlegung wird nach dem Gebrauch der Injektionsnadel aufgehoben, wodurch sich die innerhalb der Hülse vorgespannte Schraubendruckfeder entspannt und die Hülse entlang der Nadel verschiebt, wobei die freie Länge der entspannten Feder ausreicht, daß sich die Spitze der Nadel innerhalb der Hülse befindet. Wenn von außen gegen die Hülse in Richtung des Sockelteils gedrückt wird, soll eine Ausrichtung der Nadel zur Bohrung, in welcher die Nadel erneut durch die Bohrung nach außen hindurchtreten könnte, nicht mehr möglich sein.

Die der Erfindung zugrundeliegende Aufgabe besteht darin, die Schutzvorrichtung für eine Injektionsnadel der gattungsgemäßen Art so auszubilden, daß ein sicherer Schutz gegenüber Verletzungen nach dem Gebrauch gewährleistet ist.

Diese Aufgabe wird durch die Vorrichtung nach Anspruch 1 gelöst.

Wenn die Injektionsnadel in der ein Stück bildenden Schutzvorrichtung angeordnet ist, wobei sie mit dieser ein Einweg-Erzeugnis bildet, ragt die Injektionsnadel mit der für den Gebrauch erforderlichen Länge über die Schutzvorrichtung aus der Bohrung heraus. Nach der Ausführung der Injektion, wofür die Injektionsnadel sockelteilseitig beispielsweise mit einer bei Spritzen üblichen Zylinder-Kolben-Einheit verbunden werden kann, wird der Anschlagbund vom ringförmigen Fassungsteil weggezogen, wobei sich das Wendelelement dehnt. Dieses Wegziehen des Anschlagbunds erfolgt so weit, bis der Anschlagbund über die Spitze der Injektionsnadel hinaus gezogen ist. Aufgrund der Rückstellkraft des Wendelelements kann sich nun die Spitze der Injektionsnadel auf der dem Fassungsteil gegenüberliegenden Innenfläche des Anschlagbundes verhaken, so daß eine sichere Entsorgung der Injektionsnadel mit Hilfe der Schutzvorrichtung gewährleistet ist. Dadurch, daß sich die Hülse innerhalb des dehnbaren Wendelelements befindet, ergeben sich relativ geringe Abmessungen der Schutzvorrichtung in Radialrichtung, und es wird gleichzeitig unterbunden, daß die Spitze der Injektionsnadel durch die gedehnten Windungen des Wendelelements seitlich nach außen vorstehen kann.

Die Verbindung mit dem Sockelteil der Injektionsnadel erfolgt zweckmäßigerweise dadurch, daß das dehnbare Wendelelement an einem ringförmigen Fassungsteil befestigt ist, das eine Innennut für die Steckaufnahme des Sockelteils der Injektionsnadel aufweist.

Der Außendurchmesser des dehnbaren Wendelelements beträgt vorzugsweise das Vier- bis Fünffache des Außendurchmessers der Injektionsnadel.

Als Vorteil hat sich erwiesen, wenn bei ungedehntem Wendelelement die axiale Längserstreckung der Schutzvorrichtung den vierten bis fünften Teil der Gesamtlänge der Injektionsnadel beträgt.

Anhand einer Zeichnung wird ein Ausführungsbeispiel der Erfindung näher erläutert. Es zeigt:
- Fig. 1: in einer teilweise axial geschnittenen Seitenansicht eine Ausführungsform der Schutzvorrichtung mit der Injektionsnadel in Gebrauchsstellung und
- Fig. 2: in einer Ansicht wie Fig. 1 die Schutzvorrichtung mit der gebrauchten Injektionsnadel in Entsorgungsstellung.

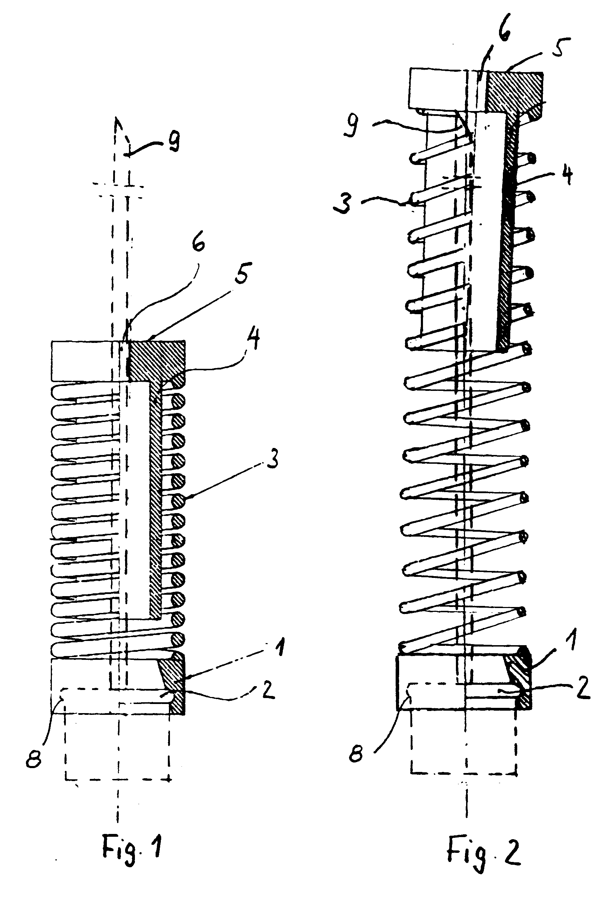

Die in Fig. 1 und 2 gezeigte Schutzvorrichtung für die Injektionsnadel 9 hat ein ringförmiges Fassungsteil 1 mit einer Innennut 2 für die Steckaufnahme eines Sockelteils 8 der Injektionsnadel 9, einen Anschlagbund 5 mit einer zentralen Bohrung 6 und ein dehnbares Wendelelement in Form einer Schraubfeder 3, die am Anschlagbund 5 und am ringförmigen Fassungsteil 1 festgelegt ist. Innerhalb der Schraubfeder 3 erstreckt sich in der in Fig. 1 gezeigten Gebrauchsstellung koaxial zu ihr und zu der Bohrung 6, jedoch außerhalb von letzterer, vom Anschlagbund 5 in Richtung des Fassungsteils 1 hin eine Hülse 4, die im Abstand vom Fassungsteil 1 endet.

Die Injektionsnadel 9 ist in der Schutzvorrichtung so angeordnet, daß ihr Sockelteil 8 in die Innennut 2 des ringförmigen Fassungsteils 1 einrastet, wobei sich die Injektionsnadel 9 im Schiebesitz durch die Bohrung 6 über die Schutzvorrichtung soweit hinaus erstreckt, daß die Injektion ohne Beeinträchtigung durch die Schutzvorrichtung ausgeführt werden kann. Der Schnappsitz zwischen dem Sockelteil 8 der Injektionsnadel 9 und der Innennut 2 des Fassungsteils 1 ist so bemessen, daß die Kraft zur Lösung dieses Schnappsitzes mindestens zehnmal größer ist als die für die erforderliche Dehnung der Schraubenfeder 3 nötige Kraft.

Nach Durchführung der Injektion, wofür eine nicht gezeigte Zylinder-Kolben-Einheit mit dem Sockelteil 8 verbunden wird, wird der Anschlagbund 5 längs der Injektionsnadel 9 unter Dehnung der Schraubenfeder 3 verschoben, bis der Eingriff zwischen der Injektionsnadel 9 und der Bohrung 6 aufgehoben ist, die Injektionsnadel 9 sich also im Inneren der Hülse 4 befindet. Durch leichtes Kippen des Anschlagbundes 5 kommt die Spitze der Injektionsnadel 9 mit der dem Fassungsteil 1 gegenüberliegenden Innenfläche des Anschlagbundes 5 in Kontakt. Nach Freigeben des Anschlagbundes 5 drückt sich die Spitze der Injektionsnadel 9 durch die Zugkraft der gedehnten Schraubenfeder 3 in die Innenfläche des Anschlagbundes 5 und verhakt sich dort, so daß eine sichere Entsorgung der Spritze 8 mit der Injektionsnadel 9 durch die Schutzvorrichtung gewährleistet ist. Die Hülse 4 verhindert dabei, daß bei zu starkem Kippen des Anschlagbundes 5 die Spitze der Injektionsnadel 9 durch die gedehnten Windungen der Schraubenfeder 3 nach außen ragen kann.

## Patentansprüche

1. Schutzvorrichtung für eine Injektionsnadel (9)
- mit einem Anschlagbund (5), der eine Bohrung (6) zum Durchführen der Injektionsnadel (9) aufweist,
- mit einem Wendelelement (3), das zwischen dem Anschlagbund (5) und einem ringförmigen Fassungsteil (1) befestigt ist, welches für eine Steckaufnahme des Sockelteils (8) der Injektionsnadel (9) ausgebildet ist, wobei der Innendurchmeser des Wendelelementes (3) größer ist als der der Bohrung (6) des Anschlagbundes (5), und
- mit einer Hülse (4), die mit dem Anschlagbund (5) fest verbunden und koaxial zu dem Wendelelement (3) angeordnet ist sowie einen Innendurchmeser hat, der größer ist als der der Bohrung (6),
dadurch gekennzeichnet,
- daß die Hülse (4) innerhalb des Wendelelements (3) angeordnet ist und
- daß das Wendelelement (3) derart dehnbar ist, daß die Rückstellkraft des gedehnten Wendelelements (3) die Spitze der Injektionsnadel (9) in die Innenfläche des Anschlagbundes (5) drückt.

2. Schutzvorrichtung nach Anspruch 1, dadurch gekennzeichnet, daß das dehnbare Wendelelement (3) eine Innennut (2) für die Steckaufnahme des Sockelteils (8) der Injektionsnadel (9) aufweist.

3. Schutzvorrichtung nach Anspruch 1 oder 2, dadurch gekennzeichnet, daß der Außendurchmesser des dehnbaren Wendelelements (3) das Vier- bis Fünffache des Außendurchmessers der Injektionsnadel (9) beträgt.

4. Schutzvorrichtung nach einem der vorhergehenden Ansprüche, dadurch gekennzeichnet, daß bei ungedehntem Wendelelement (3) die axiale Längserstreckung der Schutzvorrichtung den vierten bis fünften Teil der Gesamtlänge der Injektionsnadel (9) beträgt.

## Claims

1. Protection device for an injection needle (9) with
- an impact shoulder (5) which provides a bore (6) for passing through the injection needle (9),
- a helical element (3) which is fastened between the impact shoulder (5) and a annular socket part (1) designed for snapping-in a socket part (8) of an injection needle assembly (8, 9), with the inner diameter of the helical element (3) being greater than that of the bore (6) of the impact shoulder (5), and
- a sleeve (4) which is firmly connected to the impact shoulder (5) and arranged coaxially with the helical element (3) and provides an inner diameter which is greater than that of the bore (6),
*characterized in that*
- the sleeve (4) is arranged inside the helical element (3), and
- the helical element (3) is extensible so that the retraction force of the extended helical element (3) is sufficient for anchoring the tip of the injection needle (9) into an inner surface of the impact shoulder (5).

2. Protection device of claim 1, *characterized in that* the annular socket part (1) to which there is attached the extensible helical element (3) provides an inner groove (2) for snapping-in the socket part (8) of the injection needle assembly (8, 9).

3. Protection device of claim 1 or 2, *characterized in that* the outer diameter of the extensible helical element (3) amounts to four to five outer diameters of the injection needle (9).

4. Protection device of one of the preceding claims, *characterized in that* at non-extended helical element (3), the axial longitudinal extension of the protection device amounts to 1/4 to 1/5 of the total length of the injection needle (9).

## Revendications

1. Dispositif de protection pour une aiguille à injection (9) comprenant
- un collet de butée (5) qui présente un perçage (6) pour l'introduction de l'aiguille à injection (9),
- un élément hélicoïdal (3) fixé entre le collet de butéé (5) et une douille annulaire (1), cette dernière étant conçue pour recevoir par embronchement le socle (8) de l'aiguille à injection (9), le diamètre intérieur de l'élément hélicoïdal (3) étant supérieur à celui du perçage (6) du collet de butée (5), et
- un fourreau (4) solidaire du collet de butée (5) et disposé coaxialement par rapport à l'élément hélicoïdal (3) avec un diamètre intérieur qui est supérieur à celui du perçage (6)
caractérisé en ce que
- le fourreau (4) est disposé à l'intérieur de l'élément hélicoïdal (3) et que
- l'élément hélicoïdal (3) est extensible de telle manière que la force de rappel de l'élément hélicoïdal en extension (3) pousse la pointe de l'aiguille à injection (9) dans la face interne du collet de butée (5).

2. Dispositif de protection selon la revendication 1, caractérisé en ce que l'élément hélicoïdal extensible (3) présente une rainure intérieure (2) permettant de recevoir par embrochement le socle (8) de l'aiguille à injection (9).

3. Dispositif de protection selon l'une quelconque des revendications 1 et 2, caractérisé en ce que le diamètre extérieur de l'élément hélicoïdal extensible (3) est quatre à cinq fois supérieur au diamètre extérieur de l'aiguille à injection (9).

4. Dispositif de protection selon l'une quelconque des revendications précédentes, caractérisé en ce que, lorsque l'élément hélicoïdal n'est pas en extension, l'extension axiale du dispositif de protection fasse 1/4 jusqu'à 1/5 de la longueur totale de l'aiguille à injection.
